(19) Europäisches Patentamt
European Patent Office
Office européen des brevets

(11) Veröffentlichungsnummer: **0 079 855**
**A1**

(12) # EUROPÄISCHE PATENTANMELDUNG

(21) Anmeldenummer: **82810476.0**

(22) Anmeldetag: **08.11.82**

(51) Int. Cl.³: **C 07 C 149/40**
C 09 K 15/14, C 08 K 5/36

(30) Priorität: **12.11.81 US 320247**

(43) Veröffentlichungstag der Anmeldung:
**25.05.83 Patentblatt 83/21**

(84) Benannte Vertragsstaaten:
**BE DE FR GB IT NL**

(71) Anmelder: **CIBA-GEIGY AG**
**Patentabteilung Postfach**
**CH-4002 Basel(CH)**

(72) Erfinder: **Spivack, John D.**
**1 Blue Jay Street**
**Spring Valley New York 10977(US)**

(54) **Alkylierte Hydroxyphenylthioalkansäureester.**

(57) Die Titelverbindungen der Formel

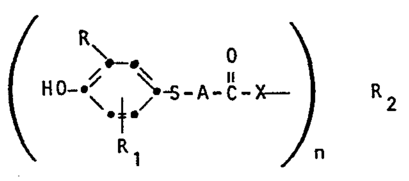

sind als Stabilisatoren für organische Polymere und Schmieröle verwendbar, um den Abbauwirkungen von Hitze, Licht und Luft entgegenzuwirken.

EP 0 079 855 A1

3-13652/+

Alkylierte Hydroxyphenylthioalkansäureester

Organische polymere Materialien wie Kunststoffe und Harze sowie Schmier- und Mineralöle unterliegen Abbau durch Wärme, Oxydation und Licht. Zahlreiche unterschiedliche Stabilisatoren zum Stabilisieren verschiedener Substrate sind dem Stand der Technik bekannt. Deren Wirksamkeit variiert je nach den Ursachen des Abbaus und dem stabilisierten Substrat. Im allgemeinen ist es schwierig, vorherzusagen, welcher Stabilisator für ein bestimmtes Anwendungsgebiet am wirksamsten und wirtschaftlichsten ist. Beispielsweise kann die Wirksamkeit des Stabilisators zur Verringerung der Flüchtigkeit davon abhängen, dass man eine Spaltung von Bindungen im Substratmolekül verhindert. Die Begrenzung der Versprödung und Beibehaltung der Elastizität in einem Polymer oder Kautschuk kann es erforderlich machen, übermässige Vernetzung und/oder Kettenspaltung zu verhindern. Die Verhütung von Verfärbung kann Hemmreaktionen erfordern, die neue Chromophore oder Farbkörper im Substrat oder Stabilisator ergeben. Probleme mit der Verarbeitungsstabilität und Unverträglichkeit sind ebenfalls in Betracht zu ziehen.

Es wurde nun gefunden, dass die Mercaptophenolderivate dieser Erfindung eine ungewöhnliche Kombination erwünschter Eigenschaften besitzen, was sie als Stabilisatoren besonders wirksam und nützlich macht. Die Verbindungen sind besonders wirksam zum Schutz von hochschlagzähem Polystyrol, Kautschukarten wie Polybutadien und Styrol/Butadienkautschuk sowie weiteren Elastomeren, worin die Beibehaltung der Elastizität und die Hemmung der Vernetzung, Rissbildung,

Verfärbung, Geruchsbildung sowie des Ausschwitzens grundlegende Erfordernisse darstellen.

Eine Anzahl von Mercaptophenolderivaten ist schon bekannt geworden. Die japanische Offenlegung 74 72 229 offenbart Ester der 3,5-Di-tert.-butyl-4-hydroxyphenylthioessigsäure und erwähnt speziell den Pentaerythrit-tetraester und den Methylester. Diesen Verbindungen wird Verwendbarkeit als Antioxydans zugeschrieben. Die japanische Offenlegung 74 75 551 beschreibt 4-Cyan- und -Alkoxycarbonyläthylthio-2,6-di-tert.-butylphenole, wobei die 4-Cyanverbindung und das Methylpropionat speziell erwähnt werden. Die in Chemical Abstracts 86, 5066m (1977) referierte russische Veröffentlichung offenbart ebenfalls Derivate des 3,5-Di-tert.-butyl-4-hydroxythiophenols. Unter den speziell genannten Verbindungen finden sich unter anderem die Methylacetat- und Methylpropionatester. Eine Wirksamkeit als Antioxydans wird diesen Verbindungen zugeschrieben. Schliesslich sind 2-(3,5-Di-tert.-butyl-4-hydroxyphenyl)-1-thioalkansäuren und deren Derivate aus der U.S. Patentschrift Nr. 4 012 523 bekannt. Niederalkylester und α,γ-substituierte Essigsäuren sind als Beispiele angegeben. Die Verbindungen der U.S. Patentschrift erscheinen auch im Journal of Medicinal Chemistry, 20, 1007-1013 (1977), wo sie wegen ihrer pharmazeutischen Verwendbarkeit zitiert werden.

Die Hauptaufgabe dieser Erfindung besteht darin, eine Klasse von Mercaptophenolderivaten zur Verfügung zu stellen, die einen weiten Bereich verbesserter Leistungsdaten bei der Stabilisierung aufweisen.

Verschiedene weitere Aufgaben und Vorteile dieser Erfindung ergeben sich aus deren nachfolgender Beschreibung.

Die erfindungsgemässen Verbindungen entsprechen der Formel

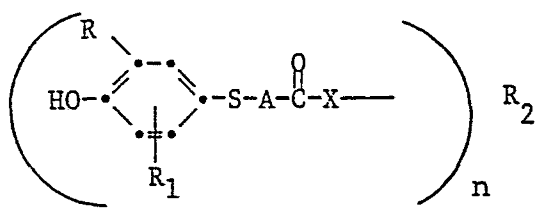

worin R für unsubstituiertes oder durch Aryl, Hydroxyaryl oder Alkaryl substituiertes Alkyl mit 1 bis 18 Kohlenstoffatomen, $R_1$ für Wasserstoff oder R, A für Alkylen mit 2 bis 12 Kohlenstoffatomen, wobei 2 bis 6 Kohlenstoffatome in der geraden Kette zwischen S und $\overset{\overset{O}{\|}}{C}$ sind und X für Sauerstoff oder Schwefel stehen, n 1 bis 6 ist und $R_2$ bei n = 1 Alkyl mit 12 bis 30 Kohlenstoffatomen, Phenyl oder niederalkylsubstituiertes Benzyl und bei n = 2 bis 6 Alkylen mit 2 bis 18 Kohlenstoffatomen, Arylen, Biphenylen, hydroxysubstituiertes Arylen, Alkanpolyyl mit 3 bis 6 Kohlenstoffatomen oder Polyoxyalkylen mit 2 bis 4 Kohlenstoffatomen in der wiederkehrenden Einheit bedeutet.

Im Rahmen der obigen Konstitution werden solche Verbindungen bevorzugt, in denen R und $R_1$ sich beide in ortho-Stellung zur Hydroxylgruppe befinden.

Als R- und $R_1$-Gruppen liegt vorzugsweise geradkettiges oder verzweigtes Alkyl mit 1 bis 8 Kohlenstoffatomen vor, wie Methyl, n-Butyl, sek.-Butyl, tert.-Butyl, tert.-Pentyl, 2-Aethylhexyl, n-Octyl und 1,1,3,3-Tetramethylbutyl. Die Gruppen Methyl, tert.-Butyl, tert.-Pentyl und 1,1,3,3-Tetramethylbutyl werden besonders bevorzugt. Ferner wird es speziell bevorzugt, wie schon erwähnt, dass sich die Gruppe $R_1$ in ortho-Stellung zur Hydroxylgruppe befindet, insbesondere wenn $R_1$ für tert.-Alkyl steht.

A ist vorzugsweise die Aethylengruppe, so dass sich Propionatderivate von Mercaptophenolverbindungen ergeben. X ist vorzugsweise Sauerstoff. n ist vorzugsweise 2 - 6, insbesondere 2 - 4. $R_2$ als Alkylen enthält vorzugsweise 2 - 6 Kohlenstoffatome.

Aryl- oder Arylensubstituenten leiten sich im allgemeinen von Phenyl, Tolyl, Mesityl, Xylyl sowie 1- und 2-Naphthyl ab.

Die erfindungsgemässen Verbindungen lassen sich dadurch herstellen, dass man beispielsweise Methyl-3-(3,5-di-tert.-butyl-4-hydroxyphenyl)-thiopropionat in Gegenwart eines Protonenakzeptors mit dem entsprechenden Mono- oder

Polyalkohol umsetzt und das freigesetzte Methanol entfernt. Typische Protonenakzeptoren sind unter anderem Lithiumsalze, tertiäre Amine, Alkalimetalle, Alkali- und Erdalkalihydroxyde, -carbonate und dergleichen. Die Reaktion wird zwar im allgemeinen in der Schmelze durchgeführt, doch kann gegebenenfalls ein Lösungsmittel vorhanden sein. Dieses ist vorzugsweise aromatisch, wie Benzol, Toluol, Xylol und dergleichen. Die Reaktionstemperatur liegt im allgemeinen im Bereich von 75 bis 200°C. Bei einer weiteren Methode zur Herstellung erfindungsgemässer Verbindungen mit n = 2 bis 6 handelt es sich um die Reaktion des entsprechenden Mercaptophenols mit dem entsprechenden Polyalkanol-polyacrylat in Gegenwart des oben erwähnten Lösungsmittels und Protonenakzeptors. Eine weitere Methode zur Herstellung solcher Verbindungen lässt sich aus einer Modifikation der in U.S. Patentschrift 4 015 523 und der japanischen Offenlegung 74, 72 229 beschriebenen Arbeitsweise ableiten, wobei die Mercaptophenole mit einer ausgewählten Halogenalkansäure oder Halogenalkanoat umgesetzt werden.

Die zur Herstellung dieser Verbindungen eingesetzten Ausgangsmaterialien sind handelsüblich oder lassen sich nach bekannten Methoden herstellen. Dabei sind wiederum die Arbeitsweisen der oben angegebenen U.S.- und japanischen Patente auf die Herstellung der Hydroxyphenylthiopropionate anwendbar. Typische Alkohole sind unter anderem n-Octadecanol, Pentaerythrit, 2,2-Dimethyl-1,3-propandiol, Tetraäthylenglykol, Trimethylolpropan, 1,6-Hexandiol und dergleichen. Typische, bei der Herstellung von Hydroxyphenyl-3-thiopropionaten verwendbare $\alpha,\beta$-ungesättigte Ester sind Methylacrylat, Aethylacrylat, n-Propylacrylat, Butylmethacrylat, n-Dodecylmethacrylat, n-Octadecylacrylat, Pentaerythrit-tetraacrylat, 1,6-Hexandioldiacrylat, Trimethylolpropantriacrylat, Sorbithexaacrylat, Mannithexaacrylat, Glycerintriacrylat, Aethylenglykoldiacrylat, Diäthylenglykoldiacrylat, Triäthylenglykoldiacrylat, Tetraäthylenglykoldimethacrylat und dergleichen. Analoge Crotonatester, Isocrotonatester, Oleatester,

Elaidinsäureester und Zimtsäureester sind ebenfalls zur Herstellung der entsprechenden substituierten Hydroxyphenyl-3-thiopropionatester durch Umsetzung mit 2-Alkyl-4-mercaptophenolen verwendbar.

Erfindungsgemässe Verbindungen sind besonders wirksam bei der Stabilisierung organischer Materialien wie Kunststoffen, Polymeren und Harzen sowie mineralischen und synthetischen Flüssigkeiten wie Schmierölen, Umlaufölen und dergleichen.

Substrate, bei denen die erfindungsgemässen Verbindungen besonders nützlich sind, umfassen schlagzähes Polystyrol, Acrylnitril/Butadien/Styrol, Styrolbutadienkautschuk, Polyester und Poly-α-olefine sowie Schmieröle insbesondere solche, die sich von Mineralölen ableiten.

Allgemein umfassen die zu stabilisierenden Polymeren:

1. Polymere von Monoolefinen und Diolefinen, beispielsweise gegebenenfalls vernetztes Polyäthylen, Polypropylen, Polyisobutylen, Polybuten-1, Polymethylpenten-1, Polyisopren oder Polybutadien sowie Polymere von Cycloolefinen z.B. Cyclopenten oder Norbornen.
2. Gemische der unter 1) angegebenen Polymeren, zum Beispiel Gemische aus Polypropylen und Polyäthylen oder Polyisobutylen.
3. Copolymere aus Monoolefinen und Diolefinen miteinander oder mit anderen Vinylmonomeren, wie zum Beispiel Aethylen/Propylen, Propylen/Buten-1, Propylen/Isobutylen, Aethylen/Buten-1, Propylen/Butadien, Isobutylen/Isopren, Aethylen/Aethylacrylat, Aethylen/Alkylmethacrylate, Aethylen/Vinylacetat oder Aethylen/Acrylsäurecopolymere und deren Salze (Ionomere) sowie Terpolymere aus Aethylen und Propylen mit einem Dien wie Hexadien, Dicyclopentadien oder Aethylidennorbornen.
4. Polystyrol.
5. Statistische Copolymere des Styrols oder α-Methyl-

styrols mit Dienen oder Acrylderivaten, wie zum Beispiel Styrol/Butadien, Styrol/Acrylnitril, Styrol/Acrylmethacrylate und Styrol/Acrylnitril/Methylacrylat, hochschlagzähe Gemische aus Styrolcopolymeren und einem anderen Polymer, wie zum Beispiel aus einem Polyacrylat, einem Dienpolymer oder einem Aethylen/Propylen/Dienterpolymer, sowie Blockcopolymere des Styrols, wie zum Beispiel Styrol/Butadien/Styrol, Styrol/Isopren/Styrol, Styrol/Aethylen/Butylen/Styrol oder Styrol/Aethylen/Propylen/Styrol.

6. Pfropfcopolymere des Styrols, wie zum Beispiel Styrol auf Polybutadien, Styrol und Acrylnitril auf Polybutadien, Styrol und Alkylacrylate oder -methacrylate auf Polybutadien, Styrol und Acrylnitril auf Aethylen/Propylen/Dienterpolymeren, Styrol und Acrylnitril auf Polyacrylaten oder Polymethacrylaten, Styrol und Acrylnitril auf Acrylat/Butadiencopolymeren sowie deren Gemische mit den unter 5) angeführten Copolymeren, beispielsweise den als ABS-, MBS-, ASA- oder AES-Polymere bekannten Copolymergemischen.

7. Halogenhaltige Polymere, wie Polychloropren, chlorierte Kautschuke, chloriertes oder sulfochloriertes Polyäthylen, Polymere halogenhaltiger Vinylverbindungen, wie zum Beispiel Polyvinylchlorid, Polyvinylidenchlorid, Polyvinylfluorid und Polyvinylidenfluorid, sowie deren Copolymere wie zum Beispiel Vinylchlorid/Vinylidenchlorid, Vinylchlorid/Vinylacetat oder Vinylidenchlorid/Vinylacetatcopolymere.

8. Sich von $\alpha,\beta$-ungesättigten Säuren und deren Abkömmlingen ableitende Polymere, wie Polyacrylate und Polymethacrylate, Polyacrylamide und Polyacrylnitril.

9. Copolymere aus den unter 8) erwähnten Monomeren miteinander oder mit anderen ungesättigten Monomeren, wie zum Beispiel Acrylnitril/Butadien, Acrylnitril/Alkylacrylat oder Acrylnitril/Vinylchloridcopolymere oder Acrylnitril/Alkylmethacrylat/Butadienterpolymere.

10. Sich von ungesättigten Alkoholen und Aminen oder deren Acylderivaten oder Acetalen ableitende Polymere, wie Polyvinylalkohol, Polyvinylacetat, Polyvinylstearat, Polyvinyl-

benzoat, Polyvinylmaleinat, Polyvinylbutyral, Polyallylphthalat oder Polyallylmelamin.

11. Homopolymere und Copolymere cyclischer Aether, wie Polyalkylenglykole, Polyäthylenoxyd, Polypropylenoxyd oder deren Copolymere mit Bis-glycidyläthern.

12. Polyacetale, wie Polyoxymethylen sowie Aethylenoxyd als Comonomer enthaltende Polyoxymethylene.

13. Polyphenylenoxyde und -sulfide.

14. Sich von Polyäthern, Polyestern oder Polybutadienen mit endständigen Hydroxylgruppen einerseits und aliphatischen oder aromatischen Polyisocyanaten andererseits ableitende Polyurethane sowie deren Vorprodukte.

15. Sich von Diaminen und Dicarbonsäuren und/oder von Aminocarbonsäuren oder den entsprechenden Lactamen ableitende Polyamide und Copolyamide, wie Polyamid-4, Polyamid-6, Polyamid-6/6, Polyamid-6/10, Polyamid 11, Polyamid 12, Poly-2,4,4-trimethylhexamethylenterephthalamid oder Poly-m-phenylenisophthalamid, sowie deren Copolymere mit Polyäthern wie zum Beispiel mit Polyäthylenglykol, Polypropylenglykol oder Polytetramethylenglykolen.

16. Polyharnstoffe, Polyimide und Polyamid-imide.

17. Sich von Dicarbonsäuren und Dialkoholen und/oder von Hydroxycarbonsäuren oder den entsprechenden Lactonen ableitende Polyester, wie Polyäthylenterephthalat, Polybutylenterephthalat, Poly-1,4-dimethylolcyclohexanterephthalat und Polyhydroxybenzoate sowie sich von Polyäthern mit Hydroxylendgruppen ableitende Copolyäther-ester.

18. Polycarbonate.

19. Polysulfone und Polyäthersulfone.

20. Vernetzte, sich von Aldehyden einerseits und Phenolen, Harnstoffen und Melaminen andererseits ableitende Polymere, wie Phenol/Formaldehydharze, Harnstoff/Formaldehydharze und Melamin/Formaldehydharze.

21. Trocknende und nicht-trocknende Alkydharze.

22. Sich von Copolyestern gesättigter und ungesättigter Dicarbonsäuren mit mehrwertigen Alkoholen und Vinylverbindungen als Vernetzungsmitteln ableitende ungesättigte

Polyesterharze und auch deren halogenhaltige, schwer entflammbare Modifikationen.

23. Sich von substituierten Acrylestern ableitende, heisshärtende Acrylharze, wie Epoxi-acrylate, Urethan-acrylate oder Polyester-acrylate.

24. Alkydharze, Polyesterharze oder Acrylatharze im Gemisch mit Melaminharzen, Harnstoffharzen, Polyisocyanaten oder Epoxidharzen als Vernetzungsmittel.

25. Sich von Polyepoxiden, beispielsweise von Bis-glycidyläthern oder cycloaliphatischen Diepoxiden und aromatischen Diepoxiden ableitende, vernetzte Epoxidharze.

26. Natürliche Polymere, wie Cellulose, Kautschuk, Gelatine und deren chemisch modifizierte polymerhomologe Derivate, wie Celluloseacetate, Cellulosepropionate und Cellulosebutyrate sowie Celluloseäther, wie Methylcellulose.

27. Natürlich vorkommende und synthetische organische Materialien, die reine monomere Verbindungen oder Gemische solcher Verbindungen darstellen, beispielsweise Mineralöle, tierische und pflanzliche Fette, Oele und Wachse, oder Oele, Fette und Wachse auf Grundlage synthetischer Ester (z.B. Phthalate, Adipate, Phosphate oder Trimellithate) sowie Gemische synthetischer Ester mit Mineralölen in beliebigem Gewichtsverhältnis, wobei diese Materialien als Weichmacher für Polymere oder als Textilspinnöle verwendbar sind, sowie wässrige Emulsionen solcher Materialien.

28. Wässrige Emulsionen natürlichen oder synthetischen Kautschuks, z.B. natürlicher Latex oder Latices carboxylierter Styrol/Butadiencopolymerer.

Im allgemeinen werden die erfindungsgemässen Stabilisatoren in Mengen von etwa 0.01 bis etwa 5 Gew.-% der stabilisierten Zusammensetzung eingesetzt, doch variiert dies mit dem jeweiligen Substrat und Anwendungszweck. Ein vorteilhafter Bereich liegt zwischen etwa 0.05 und etwa 2%, und insbesondere 0.1 und etwa 1%.

Die erfindungsgemässen Stabilisatoren lassen sich in die organischen Polymeren nach herkömmlichen Methoden in irgendeiner zweckmässigen Stufe vor der Herstellung von

Formkörpern aus diesen leicht einarbeiten. Beispielsweise kann man den Stabilisator mit dem Polymer in trockener Pulverform vermischen, oder eine Suspension oder Emulsion des Stabilisators kann in eine Lösung, Suspension oder Emulsion des Polymeren eingemischt werden. Gegebenenfalls können die erfindungsgemässen, stabilisierten Polymermassen ferner verschiedene herkömmliche Zusatzstoffe enthalten, wie die folgenden:

1. Antioxydantien

1.1. Alkylierte Monophenole, zum Beispiel 2,6-Di-tert.-butyl-4-methylphenol, 2-tert.-Butyl-4,6-dimethylphenol, 2,6-Di-tert.-butyl-4-äthylphenol, 2,6-Di-tert.-butyl-4-n-butylphenol, 2,6-Di-tert.-butyl-4-i-butylphenol, 2,6-Dicyclopentyl-4-methylphenol, 2-(α-Methylcyclohexyl)-4,6-dimethylphenol, 2,6-Di-octadecyl-4-methylphenol, 2,4,6-Tricyclohexylphenol und 2,6-Di-tert.-butyl-4-methoxymethylphenol.

1.2. Alkylierte Hydrochinone, zum Beispiel 2,6-Di-tert.-butyl-4-methoxyphenol, 2,5-Di-tert.-butyl-hydrochinon, 2,5-Di-tert.-amyl-hydrochinon und 2,6-Diphenyl-4-octadecyloxyphenol.

1.3. Hydroxylierte Thiodiphenyläther, zum Beispiel 2,2'-Thio-bis-(6-tert.-butyl-4-methylphenol), 2,2'-Thio-bis-(4-octylphenol), 4,4'-Thio-bis-(6-tert.-butyl-3-methylphenol) und 4,4'-Thio-bis-(6-tert.-butyl-2-methylphenol).

1.4 Alkyliden-bisphenole, zum Beispiel 2,2'-Methylen-bis-(6-tert.-butyl-4-methylphenol), 2,2'-Methylen-bis-(6-tert.-butyl-4-äthylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.-butylphenol), 2,2'-Aethyliden-bis-(4,6-di-tert.-butylphenol), 2,2'-Aethyliden-bis-(6-tert.-butyl-4-isobutylphenol), 4,4' Methylen-bis-(2,6-di-tert.-butylphenol), 4,4'-Methylen-bis-(6-tert.-butyl-2-methylphenol), 1,1-Bis-(5-tert.-butyl-4-hydroxy-2-methylphenyl)-butan, 2,6-Di-(3-tert.-butyl-5-methyl-2-hydroxybenzyl)-4-methylphenol, 1,1,3-Tris-(5-tert.-butyl-4-hydroxy-2-methylphenyl)-butan, 1,1-Bis-(5-tert.-butyl-4-hydroxy-2-methylphenyl)-3-n-dodecylmercapto-butan, Aethylenglykol-bis-[3,3-bis-(3'-tert.-butyl-4'-hydroxyphenyl)-butyrat], Di-(3-tert.-butyl-4-hydroxy-5-methylphenyl)-dicyclopentadien und Di-[2-(3'-tert.-butyl-2'-hydroxy-5'-methyl-benzyl)-6-tert.-butyl-4-methylphenyl]-terephthalat.

1.5. Benzylverbindungen, zum Beispiel 1,3,5-Tri-(3,5-di-tert.-butyl-4-hydroxybenzyl)-2,4,6-trimethylbenzol, Di-(3,5-di-tert.-butyl-4-hydroxybenzyl)-sulfid, 3,5-Di-tert.-butyl-4-hydroxybenzyl-mercaptoessigsäureisooctylester, Bis-(4-tert.-butyl-3-hydroxy-2,6-dimethylbenzyl)-dithiol-terephthalat, 1,3,5-Tris-(3,5-di-tert.-butyl-4-hydroxy-benzyl)-isocyanurat, 1,3,5-Tris-(4-tert.-butyl-3-hydroxy-2,6-dimethylbenzyl)-isocyanurat, 3,5-Di-tert.-butyl-4-hydroxybenzyl-phosphorsäuredioctadecylester und das Calciumsalz des 3,5-Di-tert.-butyl-4-hydroxybenzyl-phosphorsäuremonoäthylesters.

1.6. Acylaminophenole, zum Beispiel 4-Hydroxy-laurinsäureanilid, 4-Hydroxy-stearinsäureanilid und 2,4-Bis-octylmercapto-6-(3,5-di-tert.-butyl-4-hydroxyanilino)-s-triazin.

1.7. Ester der β-(3,5-Di-tert.-butyl-4-hydroxyphenyl)-propionsäure mit einwertigen oder mehrwertigen Alkoholen, zum Beispiel Methanol, Octadecanol, 1,6-Hexandiol, Neopentylglykol, Thiodiäthylenglykol, Diäthylenglykol, Triäthylenglykol, Pentaerythrit, Tris-hydroxyäthylisocyanurat und Di-hydroxyäthyloxalsäurediamid.

1.8. Ester der β-(5-tert.-Butyl-4-hydroxy-3-methylphenyl)-propionsäure mit einwertigen oder mehrwertigen Alkoholen, zum Beispiel Methanol, Octadecanol, 1,6-Hexandiol, Neopentylglykol, Thiodiäthylenglykol, Diäthylenglykol, Triäthylenglykol, Pentaerythrit, Tris-hydroxyäthylisocyanurat und Di-hydroxyäthyloxalsäurediamid.

1.9. Amide der β-(3,5-Di-tert.-butyl-4-hydroxyphenyl)-propionsäure, zum Beispiel N,N'-Di-(3,5-di-tert.-butyl-4-hydroxyphenylpropionyl)-hexamethylendiamin, N,N'-Di-(3,5-di-tert.-butyl-4-hydroxyphenylpropionyl)-trimethylendiamin

und N,N'-Di-(3,5-di-tert.-butyl-4-hydroxyphenylpropionyl)-hydrazin.

2. UV-Absorber und Lichtschutzmittel

2.1. 2-(2'-Hydroxyphenyl)-benztriazole, zum Beispiel das 5'-Methyl-, 3',5'-Di-tert.-butyl-, 5'-tert.-Butyl-, 5'-(1,1,3,3-Tetramethylbutyl)-, 5-Chlor-3',5'-di-tert.-butyl-, 5-Chlor-3'-tert.-butyl-5'-methyl-, 3'-sek.-Butyl-5'-tert.-butyl- und 4'-Octoxy-3',5'-di-tert.-amylderivat.

2.2. 2-Hydroxy-benzophenone, zum Beispiel das 4-Hydroxy-, 4-Methoxy-, 4-Octoxy-, 4-Decyloxy-, 4-Dodecyloxy-, 4-Benzyloxy-, 4,2',4'-Trihydroxy- und 2'-Hydroxy-4,4'-dimethoxyderivat.

2.3. Ester gegebenenfalls substituierter Benzoesäuren, zum Beispiel Phenylsalicylat, 4-tert.-Butyl-phenylsalicylat, Octylphenylsalicylat, Dibenzoylresorcin, Bis-(4-tert.-butylbenzoyl)-resorcin, Benzoylresorcin und 3,5-Di-tert.-butyl-4-hydroxybenzoesäure-2,4-di-tert.-butylphenylester.

2.4. Acrylate, zum Beispiel α-Cyan-β,β-diphenylacrylsäure-äthylester oder -isooctylester, α-Carbomethoxyzimtsäure-methylester, α-Cyan-β-methyl-p-methoxyzimtsäuremethylester oder -butylester, α-Carbomethoxy-p-methoxyzimtsäuremethylester und N-(β-Carbomethoxy-β-cyanvinyl)-2-methylindolin.

2.5. Nickelverbindungen, zum Beispiel Nickelkomplexe des 2,2'-Thio-bis-[4-(1,1,3,3-tetramethylbutyl)-phenols], wie der 1:1- oder 1:2-Komplex, gegebenenfalls mit weiteren Liganden wie n-Butylamin, Triäthanolamin oder N-Cyclohexyl-diäthanolamin, Nickeldibutyldithiocarbamat, Nickelsalze von 4-Hydroxy-3,5-di-tert.-butylbenzylphosphonsäuremono-alkylestern, wie der Methyl-, Aethyl - oder Butylester, Nickelkomplexe von Ketoximen wie 2-Hydroxy-4-methylphenyl-undecyl-ketonoxim und Nickelkomplexe des 1-Phenyl-4-lauroyl-5-hydroxypyrazols, gegebenenfalls mit weiteren Liganden.

2.6. Sterisch gehinderte Amine, zum Beispiel Bis-(2,2,6,6-tetramethylpiperidyl)-sebacinat, Bis-(1,2,2,6,6-pentamethylpiperidyl)-sebacinat, n-Butyl-3,5-di-tert.-butyl-4-hydroxybenzylmalonsäure-bis-(1,2,2,6,6-pentamethylpiperidyl)-ester, das Kondensationsprodukt aus 1-Hydroxyäthyl-2,2,6,6-

tetramethyl-4-hydroxypiperidin und Bernsteinsäure, das Kondensationsprodukt aus N,N'-(2,2,6,6-Tetramethylpiperidyl)-hexamethylendiamin und 4-tert.-Octylamino-2,6-dichlor-1,3,5-s-triazin und Tris-(2,2,6,6-tetramethylpiperidyl)-nitrilotriacetat.

2.7. Oxalsäurediamide, zum Beispiel 4,4'-Dioctyloxy-oxanilid, 2,2'-Dioctyloxy-5,5'-di-tert.-butyl-oxanilid, 2,2'-Didodecyloxy-5,5'-di-tert.-butyl-oxanilid, 2-Aethoxy-2'-äthyl-oxanilid, N,N'-Bis-(3-dimethylaminopropyl)-oxalamid, 2-Aethoxy-5-tert.-butyl-2'-äthyl-oxanilid und dessen Gemisch mit 2-Aethoxy-2'-äthyl-5,4'-di-tert.-butyl-oxanilid und Gemische aus ortho- und para-methoxy- sowie o- und p-äthoxydisubstituierten Oxaniliden.

3. Metalldesaktivatoren, zum Beispiel N,N'-Diphenyloxalsäurediamid, N-Salicylal-N'-salicyloylhydrazin, N,N'-Bis-salicyloylhydrazin, N,N'-Bis-(3,5-di-tert.-butyl-4-hydroxyphenylpropionyl)-hydrazin, 3-Salicyloylamino-1,2,4-triazol und Bis-benzylidenoxalsäuredihydrazid.

4. Phosphite und Phosphonite, zum Beispiel Triphenylphosphit, Diphenylalkylphosphite, Phenyldialkylphosphite, Tri-(nonylphenyl)-phosphit, Trilaurylphosphit, Trioctadecylphosphit, Distearyl-pentaerythrit-diphosphit, Tris-(2,4-di-tert.-butylphenyl)-phosphit, Diisodecyl-pentaerythrit-diphosphit, Di-(2,4-di-tert.-butylphenyl)-pentaerythrit-diphosphit, Tristearyl-sorbit-triphosphit und Tetrakis-(2,4-di-tert.-butylphenyl)-4,4'-biphenylylen-diphosphonit.

5. Peroxydzerstörende Verbindungen, zum Beispiel Ester der β-Thiodipropionsäure, wie der Lauryl-, Stearyl-, Myristyl- oder Tridecylester, Mercaptobenzimidazol oder das Zinksalz des 2-Mercaptobenzimidazols, Zink-dibutyl-dithiocarbamat, Dioctadecyldisulfid und Pentaerythrit-tetrakis-(β-dodecylmercapto)-propionat.

6. Polyamidstabilisatoren, zum Beispiel Kupfersalze in Kombination mit Jodiden und/oder Phosphorverbindungen und Salzen des zweiwertigen Mangans.

7. Basische Costabilisatoren, zum Beispiel Melamin, Polyvinylpyrrolidon, Dicyandiamid, Triallylcyanurat, Harn-

stoffderivate, Hydrazinderivate, Amine, Polyamide, Polyurethane, Alkali- und Erdalkalisalze höherer Fettsäuren, z.B. Ca-Stearat, Zn-Stearat, Mg-Stearat, Na-Ricinoleat und K-Palmitat, und Antimon- oder Zink-brenzkatechinat.

8. Nukleierungsmittel, zum Beispiel 4-tert.-Butylbenzoesäure, Adipinsäure und Diphenylessigsäure.

9. Füllstoffe und Verstärkungsstoffe, zum Beispiel Calciumcarbonat, Silicate, Glasfasern, Asbest, Talk, Kaolin, Glimmer, Bariumsulfat, Metalloxyde und -hydroxyde, Russ und Graphit.

10. Weitere Zusatzstoffe, zum Beispiel Weichmacher, Schmierstoffe, Emulgatoren, Pigmente, optische Aufheller, Flammschutzmittel, Antistatika und Treibmittel.

Die erfindungsgemässen Verbindungen sind für sich als einziger Stabilisator mit entweder hauptsächlich einer Antioxydansfunktion oder einer Lichtschutzfunktion verwendbar, oder der Stabilisator kann in sich den Nutzen eines Antioxydans und eines Lichtschutzmittels vereinigen.

Die vorliegenden Verbindungen lassen sich zwar nutzbringend als Stabilisatoren für verschiedene Substrate sowohl für sich als auch zusammen mit weiteren Zusatzstoffen verwenden, doch verleiht eine Kombination der vorliegenden Verbindungen mit ausgewählten sterisch gehinderten Phenolantioxydantien solchen Substraten erhöhten Schutz. Als Phenolantioxydantien haben sich solche aus der Gruppe 2,6-Di-tert.-butyl-4-methylphenol, 4,4'-Thio-bis-(6-tert.-butyl-3-methylphenol), 2,2'-Methylen-bis-(6-tert.-butyl-3-methylphenol), 4,4'-Methylen-bis-(2,6-di-tert.-butylphenol), 1,1,3-Tris-(5-tert.-butyl-4-hydroxy-2-methylphenyl)-butan, 1,3,5-Tris-(3,5-di-tert.-butyl-4-hydroxybenzyl)-2,4,6-trimethylbenzol, 2-Octylthio-4,6-bis-(3,5-di-tert.-butyl-4-hydroxyanilino)-s-triazin, n-Octadecyl-3,5-di-tert.-butyl-4-hydroxyhydrocinnamat, Neopentantetrayl-tetrakis-(3,5-di-tert.-butyl-4-hydroxyhydrocinnamat), 1,3,5-Tris-(3,5-di-tert.-butyl-4-hydroxybenzyl)-isocyanurat, Thiodiäthylen-bis-(3,5-di-tert.-butyl-4-hydroxyhydrocinnamat), der Tris-(2-hydroxyäthyl)-isocyanuratester

der β-(3,5-Di-tert.-butyl-4-hydroxyphenyl)-propionsäure, 6,6'-Aethyliden-bis-(2,4-di-tert.-butylphenol), 6,6-Methylen-bis-(2,4-di-tert.-butylphenol) und 1,3,5-Tris-(4-tert.-butyl-3-hydroxy-2,6- dimethylbenzyl)-isocyanurat besonders bewährt.

Die Zusammensetzungen bestehen beispielsweise aus (a) einem Substrat, vorzugsweise schlagzähem Polystyrol oder den anderen oben erwähnten bevorzugten Substraten, (b) etwa 0.01 bis etwa 5 Gew.-%, bezogen auf die Zusammensetzung, und vorzugsweise etwa 0.025 bis etwa 2% sowie besonders bevorzugt 0.025 bis 1%, einer erfindungsgemässen Verbindung oder deren Gemisch sowie gegebenenfalls (c) einem phenolischen Antioxydans oder einem Gemisch solcher Antioxydantien aus der eben angeführten Gruppe, ebenfalls im Bereich von 0.01 bis 5 Gew.-% und vorzugsweise 0.05 bis 1 Gew.-% bezogen auf die Zusammensetzung.

In ähnlicher Weise werden die folgenden Lichtschutzmittel, entweder für sich oder in Verbindung mit den angeführten Phenolantioxydantien, als Zusatzstoffe zum Einbau mit den vorliegenden Stabilisatoren in die angegebenen Substrate bevorzugt verwendet: 2-(2'-Hydroxy-5'-methylphenyl)-benztriazol, 2-(3',5'-Di-tert.-butyl-2'-hydroxyphenyl)-benztriazol, 2-(2'-Hydroxy-3',5'-di-tert.-amylphenyl)-benztriazol, Nickel-bis-[O-äthyl-(3,5-di-tert.-butyl-4-hydroxybenzyl)]-phosphonat, Bis-(1,2,2,6,6-pentamethyl-4-piperidyl)-2-n-butyl-2-(3,5-di-tert.-butyl-4-hydroxybenzyl)-malonat, Bis-(2,2,6,6-tetramethyl-4-piperidyl)-sebacinat, ein Polymer aus Bernsteinsäuredimethylester und 4-Hydroxy-2,2,6,6-tetramethyl-1-piperidinäthanol sowie ein Polymer aus 2,4-Dichlor-6-octylamino-s-triazin und N'-(2,2,6,6-Tetramethyl-4-piperidyl)-hexamethylendiamin.

Die nachfolgenden Beispiele dienen der weiteren Erläuterung der vorliegenden Erfindung. Dabei sind Teile stets Gewichtsteile, falls nicht anders angegeben.

<u>Beispiel 1</u>: <u>Pentaerythrit-tetrakis-[3-(3,5-di-tert.-butyl-4-hydroxyphenylthio)-propionat]</u>

Man legt 42,8 g Methyl-3-(3,5-di-tert.-butyl-

hydroxyphenylthio)-propionat (0,132 Mol), 4,17 g Pentaerythrit (0,03 Mol) und 0,137 g Lithiumamid (0,006 Mol) unter Stickstoff in einem Kolben vor, verschmilzt die Komponenten und erhitzt die Schmelze unter Rühren von einer Temperatur von anfänglich 130°C unter Atmosphärendruck auf eine Endtemperatur von 160°C bei 15 mm Hg Druck.

10 g-Anteile des Rohprodukts werden in 100 ml Toluol/Acetonitril (98:2) gelöst und durch Entspannungschromatographie gereinigt, indem man sie durch eine 10 cm-Säule mit Füllkörpern bis zu einer Höhe von 13 cm leitet. Diese Arbeitsweise wird viermal wiederholt. Das gesamte gereinigte Material wird dann aus 2-Propanol kristallisiert, was weisse, bei 113 bis 118°C schmelzende Kristalle liefert.

| Analyse | %C | %H |
|---|---|---|
| berechnet: | 67,1 | 8,3 |
| gefunden: | 67,4 | 8,3 |

Beispiel 2: 2,2-Dimethyl-1,3-propandiol-bis-[3-(3,5-di-tert.-butyl-4-hydroxyphenylthio)-propionat]

Man verschmilzt 9,73 g Methyl-3-(3,5-di-tert.-butyl-4-hydroxyphenylthio)-propionat (0,03 Mol), 1,60 g 2,2-Dimethyl-1,3-propandiol (0,015 Mol) und 0,022 g Lithiumhydroxyd (0,003 Mol) unter Stickstoff und rührt 2 3/4 Stunden bei 114°C bei Atmosphärendruck unter Auffangen des freigesetzten Methanols. Man erhitzt noch 2 Stunden bei 120°C unter Atmosphärendruck und schliesslich 3 3/4 Stunden bei 120 bis 131°C unter 15 mm Hg Druck. Das Reaktionsgemisch wird in 50 ml Toluol mit einem Tropfen Essigsäure aufgelöst. Die Toluollösung wäscht man nacheinander mit Wasser, gesättigter Natriumbicarbonatlösung, Wasser und gesättigter Kochsalzlösung, trocknet mit Natriumsulfat und entfernt dann das Toluol durch Destillation bei vermindertem Druck. Das gewonnene Produkt wird zunächst aus einem 2-Propanol/Wassergemisch und dann aus 2-Propanol kristallisiert. Man erhält das Produkt als weisse Kristalle mit einem Schmelzpunkt von 76-79°C nach Trocknung bei vermindertem Druck.

| Analyse: | %C | %H |
|---|---|---|
| berechnet: | 68,0 | 8,7 |
| gefunden: | 67,8 | 8,9 |

Beispiel 3: n-Octadecyl-3-(3,5-di-t-butyl-4-hydroxyphenylthio)-propionat

Die Verbindung in diesem Beispiel wird nach einer ähnlichen Arbeitsweise wie in Beispiel 2 hergestellt, unter Ersatz des 2,2-Dimethyl-1,3-propandiols durch n-Octadecanol und Umsetzen mit äquimolaren Anteilen Methyl-3-(3,5-di-tert.-butyl-4-hydroxyphenylthio)-propionat für 5 Stunden bei 118-120$^{o}$ und dann für 3 1/4 Stunden bei 125$^{o}$C unter Auffangen des freigesetzten Methanols. Die Schmelze wird mit Toluol verdünnt und nacheinander mit Wasser neutral und schliesslich mit gesättigtem Natriumbicarbonat und Wasser gewaschen. Nach Trocknung über Natriumsulfat wird die klare Lösung durch Vakuumdestillation vom Toluol befreit und der Rückstand aus einem Acetonitril/Chloroformlösungsmittelgemisch umkristallisiert. Nach Trocknen schmelzen die weissen Kristalle bei 30 bis 33$^{o}$C.

| Analyse: | %C | %H |
|---|---|---|
| berechnet: | 74,7 | 11,1 |
| gefunden: | 74,7 | 11,2 |

Beispiel 4: 1,6-Hexandiol-bis-[(3,5-di-tert.-butyl-4-hydroxyphenylthio)-propionat]

Man gibt 3,38 g in 10 ml Toluol gelöstes 1,6-Hexandiol-diacrylat (0,015 Mol) im Verlauf von 5 Minuten bei 25$^{o}$C tropfenweise zu einer Lösung von 7,3 g 4-Mercapto-2,6-di-tert.-butylphenol (0,03 Mol) und 0,15 g Triäthylamin (0,0015 Mol) in 50 ml Toluol und rührt die farblose Lösung dann bei 55$^{o}$-60$^{o}$C. Nach Entfernung des Reaktionslösungsmittels durch Vakuumdestillation bei etwa 0,5 mm Hg wird der erhaltene Rückstand in 12 ml Toluol wieder aufgelöst und nacheinander mit wässriger 2 n-Natronlauge, Wasser auf pH 5, wässriger 3 n-Salzsäure und Wasser gewaschen, bis das Waschwasser einen pH von 4 bis 5 aufweist. Dann wird das Produkt über Natriumsulfat getrocknet. Nach Entfernung des Lösungsmittels durch Vakuumdestillation bis

zu einem Enddruck von etwa 0,2 mm Hg kristallisiert man den Rückstand aus Isopropanol. Man erhält das Produkt als bei 116-119°C schmelzende weisse Kristalle.

| Analyse: | %C | %H |
|---|---|---|
| berechnet: | 68,3 | 8,9 |
| gefunden: | 68,3 | 8,5 |

Beispiel 5: Tetraäthylenglykol-bis-[3-(3,5-di-tert.-butyl-4-hydroxyphenylthio)-propionat]

Die vorliegende Verbindung wird nach der Reaktionsweise von Beispiel 4 unter Ersatz des 1,6-Hexandiol-diacrylats durch Tetraäthylenglykol-diacrylat hergestellt. Man wäscht das Reaktionsgemisch nacheinander mit wässriger 2 n-Natronlauge und Wasser, wässriger 3 n-Salzsäure und Wasser, bis das Waschwasser einen pH von 4 bis 5 aufweist. Nach Trocknen über Natriumsulfat und Entfernung des Lösungsmittels durch Vakuumdestillation isoliert man das Produkt als fast farblose Flüssigkeit.

| Analyse: | %S |
|---|---|
| berechnet: | 8,2 |
| gefunden: | 8,1 |

Beispiel 6: Trimethylolpropan-tris-[3-(3,5-di-tert.-butyl-4-hydroxyphenylthio)-propionat]

Die vorliegende Verbindung wird nach der Reaktionsweise von Beispiel 4 unter Ersatz des 1,6-Hexandiol-diacrylats durch Trimethylolpropan-triacrylat sowie unter Verlängerung der Reaktionszeit auf 18 Stunden bei 70 bis 75°C hergestellt. Dann streift man das Toluol und Triäthylamin im Vakuum ab und gibt frisches Toluol dazu. Die Lösung wird dann nacheinander mit Wasser, wässriger 2 n-Natronlauge und Wasser gewaschen und danach über Natriumsulfat getrocknet. Das Toluol wird durch Vakuumdestillation (0,2 mm Hg) entfernt. Den Rückstand reinigt man durch Entspannungschromatographie, wobei man das Produkt als farblosen Sirup erhält.

| Analyse: | %C | %H |
|---|---|---|
| berechnet: | 67,7 | 8,6 |
| gefunden: | 69,0 | 8,6 |

Beispiel 7: Die vorliegende Verbindung wird unter Ersatz des 2,6-Di-tert.-butyl-4-mercaptophenols in Beispiel 4 durch 2-tert.-Butyl-4-mercapto-6-methylphenol hergestellt. Man gewinnt 1,6-Hexandiol-bis-[3-(3-tert.-butyl-5-methyl-4-hydroxyphenylthio)-propionat] als bei 101-103°C schmelzende weisse Kristalle.

Beispiel 8: Die vorliegende Verbindung wird nach der Arbeitsweise von Beispiel 4 unter Ersatz des 2,6-Di-tert.-butyl-4-mercaptophenols durch 2,6-Dimethyl-4-mercaptophenol hergestellt. Man gewinnt 1,6-Hexandiol-bis-[3-(3,5-dimethyl-4-hydroxyphenylthio)-propionat] als bei 91-94°C schmelzende weisse Kristalle.

Gemäss der Arbeitsweise der Beispiele werden die folgenden Verbindungen wie unten in der Tabelle angegeben hergestellt:

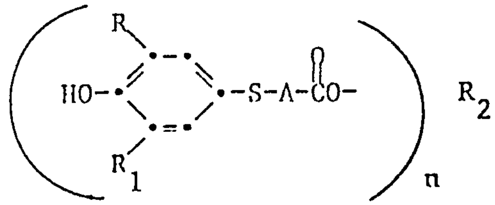

| Beispiel Nr. | R | $R_1$ | A | n | $R_2$ | Hergestellt gemäss Beispiel |
|---|---|---|---|---|---|---|
| 9 | $-CH_3$ | $-CH_3$ | $-(CH_2)_2-$ | 4 | 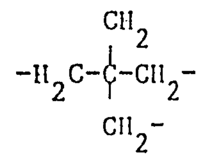 | 1 |
| 10 | $-C(CH_3)_3$ | $-CH_3$ | $-(CH_2)_2-$ | 2 | 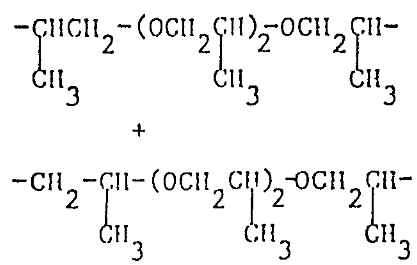 | 1 |

| Beispiel Nr. | R | $R_1$ | A | n | $R_2$ | Physik. Eigenschaften | Hergestellt gemäss Beispiel |
|---|---|---|---|---|---|---|---|
| 11 | $-C(CH_3)_3$ | $-C(CH_3)_3$ | $-(CH_2)_2-$ | 2 | 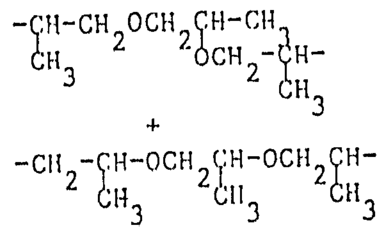 | Oel | 1 |
| 12 | $-C(CH_3)_3$ | $-CH_3$ | $-(CH_2)_2-$ | 4 | 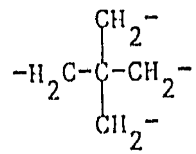 | Glas | 1 |
| 13 | $-C(CH_3)_3$ | $-C(CH_3)_3$ | 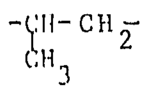 | 2 | $-(CH_2)_6-$ | Schmelzpunkt 80-94°C | 4 |
| 14 | $-C(CH_3)_3$ | $-C(CH_3)_3$ | 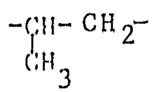 | 4 | 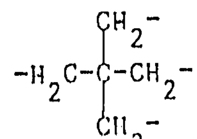 | Glas | 1 |
| 15 | $-C(CH)_3$ | $-C(CH_3)_3$ | 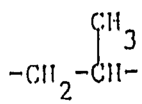 | 4 | 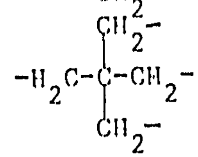 | Glas | 1 |
| 16 | $-C(CH_3)_3$ | $-C(CH_3)_3$ | 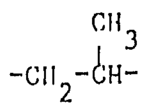 | 2 | $-(CH_2)_6-$ | Schmelzpunkt 98-106°C | 4 |

| Beispiel Nr. | R | $R_1$ | A | n | $R_2$ | Physik. Eigenschaften | Hergestellt gemäss Beispiel |
|---|---|---|---|---|---|---|---|
| 17 | $-C(CH_3)_3$ | $-CH_3$ | $-(CH_2)_2-$ | 2 | 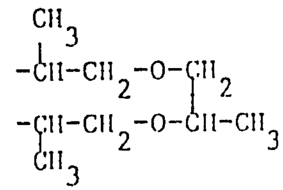 | Sirup | 1 |
| 18 | $-CH_3$ | $-CH_3$ | 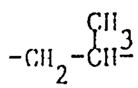 | 2 | $-(CH_2)_6-$ | Schmelzpunkt 100-104°C | 4 |
| 19 | $-C(CH_3)_3$ | $-C(CH_3)_3$ | 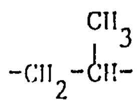 | 1 | $-nC_{18}H_{37}$ | Schmelzpunkt 32-34°C | 3 |
| 20 | $-C(CH_3)_3$ | $-C(CH_3)_3$ | $-(CH_2)_2-$ | 2 | $-CH_2-C\equiv C-CH_2-$ | Schmelzpunkt 96-99°C | 1 |
| 21 | $-CH_3$ | $-CH_3$ | 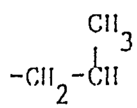 | 1 | $-nC_{18}H_{37}$ | Schmelzpunkt 49-51°C | 3 |
| 22 | $-C(CH_3)_3$ | $-C(CH_3)_3$ | 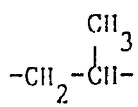 | 2 | $-(CH_2)_2-O-(CH_2)_2-O(CH_2)_2-$ | Sirup | 4 |
| 23 | $-C(CH_3)_3$ | $-C(CH_3)_3$ | $-(CH_2)_2-$ | 1 | 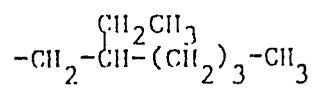 | Flüssigkeit | 3 |
| 24 | $-C(CH_3)_3$ | $-CH_3$ | 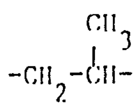 | 2 | $-(CH_2)_2-O-(CH_2)_2-O-(CH_2)_2$ | Sirup | 4 |

Beispiel 25: Dieses Beispiel veranschaulicht die stabilisierenden Eigenschaften der vorliegenden Verbindungen in schlagzähem Polystyrol.

Die entsprechenden Verbindungen werden in je 0,1% Konzentration dem Styrolmonomer vor der Polymerisation zugesetzt. Das entstandene hochschlagzähe Polystyrolharz wird gereinigt und geschnitzelt. Die Harzschnitzel werden dann auf einem 2,54 cm 24/1 = L/D-Extruder bei einer Schmelzetemperatur von 260°C extrudiert und 7 Minuten bei einer Temperatur von 163°C und einem Druck von 140 $kg/cm^2$ zu einer Folie gleichförmiger Dicke von 0,752 mm verpresst. Kontrollfolien werden auf analoge Weise hergestellt. Die Folie wird dann in ungefähr 10 x 1,25 cm grosse Streifen zerschnitten. Die Streifen werden dann bei 150°C im Ofen gealtert und von Zeit zu Zeit auf ihre prozentuale Dehnung im Instron-Tensile-Testing-Apparatus (Instron Engineering Corp., Massachusetts) geprüft.

Vergleichbare Folien werden entsprechend in 5,08 cm x 5,08 cm x 0,752 mm grosse Plättchen zerschnitten. Diese werden dann durch Bestrahlung in einem Xenotest-Apparat Typ "150" geprüft. Der Verlauf der Alterung wird durch Bestimmung des Yellowness Index (YI) nach ASTM D-1925-63T verfolgt.

Die Ergebnisse dieser Testverfahren sind in der nachfolgenden Tabelle angegeben.

Stabilisatorkonzentration - 0,1 Gew.-%

| Antioxydans | % Dehnung nach | | | | | YI nach | | | | |
|---|---|---|---|---|---|---|---|---|---|---|
| | 0 | 30 Min. | 60 Min. | 90 Min. | 120 Min. | 0 | 30 Min. | 60 Min. | 90 Min. | 120 Min. |
| Bsp. 5 | 60 | 43 | 48 | 33 | 11 | -1 | -1 | 2 | 5 | 8 |
| Bsp. 7 | 43 | 37 | 31 | 15 | 10 | -2 | 0 | 2 | 5 | 9 |
| Bsp. 8 | 26 | 17 | 14 | 7 | 7 | -3 | 0 | 5 | 8 | 13 |
| Bsp. 11* | 46 | 46 | 33 | 32 | 33 | -1 | 0 | 3 | 4 | 5 |
| Bsp. 12 | 52 | 50 | 38 | 28 | 18 | -2 | 0 | -1 | 2 | 1 |
| Bsp. 13 | 18 | 26 | 26 | 8 | 8 | -2 | 2 | 2 | 2 | 1 |
| Bsp. 14 | 38 | 18 | 24 | 10 | 8 | 0 | 4 | 6 | 9 | 11 |
| Bsp. 15 | 44 | 46 | 38 | 24 | 17 | -2 | 3 | 5 | 7 | 11 |
| Bsp. 16 | 50 | 40 | 30 | 17 | 9 | -1 | 5 | 14 | 31 | 13 |
| Bsp. 17 | 38 | 43 | 53 | 34 | 18 | 0 | 2 | 10 | 15 | 16 |
| Bsp. 18 | 50 | 23 | 23 | 9 | 7 | -1 | 3 | 11 | 15 | 18 |
| Bsp. 19 | 33 | 33 | 22 | 10 | 7 | -1 | 1 | 8 | 11 | - |
| Bsp. 20 | 47 | 29 | 23 | 20 | 8 | 4 | 8 | 17 | 20 | 22 |
| Bsp. 22 | 49 | - | 44 | 61 | 34 | -2 | 2 | 6 | 5 | 10 |
| Grundharz | 33 | 7 | 7 | 3 | 4 | 7 | 18 | 30 | 38 | 43 |

* 0,1 % Stabilisator +
0,2% Ditridecylthiodipropionat

Beispiel 26: Die unten angeführten Verbindungen werden nach der "Standard Test Method for Oxidation Stability of Steam Turbine Oils by the Rotating Bomb [Standardprüfmethode für die Oxydationsbeständigkeit von Dampfturbinenölen in der Rollbombe]", ASTM TEST Nr. 02272-67, Neuausgabe 1977, geprüft. Das Grundöl ist ein raffiniertes Mineralöl (EXXON LO 5084).

| Antioxydans | Zeit (Min.) |
|---|---|
| Beispiel 1 | 137 |
| Beispiel 2 | 160 |
| Beispiel 3 | 60 |
| Beispiel 4 | 144 |
| Beispiel 5 | 131 |
| Beispiel 6 | 122 |
| Beispiel 16 | 132 |
| Beispiel 18 | 145 |
| Beispiel 22 | 120 |
| Beispiel 24 | 108 |
| Grundöl | 31 |

Diese Daten demonstrieren somit die erhöhte Oxydationsbeständigkeit der die vorliegenden Stabilisatoren enthaltenden Prüfproben.

Beispiel 27: Eine Charge unstabilisiertes Polypropylenpulver (Hercules Profax 6501) wird mit 0,2 Gew.-% verschiedener erfindungsgemässer Verbindungen innig vermischt. Die vermischten Materialien werden dann 5 Minuten bei 182°C auf einem Zweiwalzenstuhl gewalzt; danach zieht man das stabilisierte Polypropylen als Folie vom Walzwerk ab und lässt es abkühlen.

Die gewalzten Polypropylenfolien werden dann in Stücke geschnitten und 5 Minuten in einer hydraulischen Presse bei 218°C und 19,25 $kg/cm^2$ Druck verpresst. Die erhaltenen Plättchen von 0,635 mm Dicke werden im Umlaufofen bei 150°C auf Schnellalterungsbeständigkeit untersucht. Als Versagen der Plättchen werden die ersten Anzeichen einer Zersetzung (z.B. Risse oder braune Kanten) angesehen.

Entsprechend wird die Farbe der ursprünglichen und im Ofen gealterten Proben gemäss der Standard ASTM-Gardner-Farbabtastung bewertet.

Die Ergebnisse dieser Prüfungen sind in der nachfolgenden Tabelle angeführt.

| | 0,2% Stabilisator | | 0,1% Stabilisator + 0,3% Distearylthiodipropionat | |
|---|---|---|---|---|
| Antioxydans | Zeit bis zum Versagen (Stunden) | Farbe 100 Stunden | Zeit bis zum Versagen (Stunden) | Farbe 100 Stunden |
| Grundharz | 3 | 0 | < 20 | 2 |
| Bsp. 1 | 750 | 0 | 930 | 0 |
| Bsp. 4 | 580 | 1 | 830 | 1 |
| Bsp. 12 | 310 | 2 | 640 | 1 |
| Bsp. 13 | 530 | 1 | 712 | 1 |
| Bsp. 14 | 740 | 2 | 740 | 1 |
| Bsp. 15 | 580 | 1 | 650 | 1 |
| Bsp. 16 | 410 | 1 | 650 | 1 |
| Bsp. 18 | 244 | 4 | 550 | 1 |
| Bsp. 19 | 250 | 1 | 320 | 1 |
| Bsp. 20 | 350 | 1 | 490 | 1 |
| Bsp. 21 | 250 | 1 | 410 | 1 |
| Bsp. 22 | 340 | 1 | 500 | 1 |

Beispiel 27a: Man verfährt wie in Beispiel 27 unter Verwendung von 0,2 Gew.-% Stabilisator zur Herstellung von 0,13 mm dicken Filmen. Diese werden dann bis zum Versagen in einer Höhensonne/Schwarzlichtkammer ausgesetzt. Das Versagen wird wie in Beispiel 27 bestimmt.

Die Ergebnisse sind in der nachfolgenden Tabelle angegeben.

| Antioxydans | Zeit bis zum Versagen (Stunden) |
|---|---|
| Bsp. 1 | 320 |
| Bsp. 4 | 310 |
| Bsp. 12 | 490 |
| Bsp. 13 | 440 |
| Bsp. 14 | 470 |
| Bsp. 15 | 490 |
| Bsp. 16 | 440 |
| Bsp. 18 | 370 |
| Bsp. 19 | 480 |
| Bsp. 20 | 430 |
| Bsp. 21 | 410 |
| Bsp. 22 | 520 |
| - (Kontrolle) | 300 |

Die Daten demonstrieren wiederum die ausgezeichnete Stabilisierungsleistung der vorliegenden Verbindungen.

Beispiel 28: Dieses Beispiel veranschaulicht die Stabilisierwirkung der vorliegenden Verbindungen bei der Verwendung in Faserausrüstungen.

Arbeitsweise

Man löst 2,0 Gew.-% des Stabilisators in einer aus einem Gemisch eines Pentaerythrit-tetraesters und ungesättigtem pflanzlichen Oel (von Dupont) bestehenden Faserausrüstung. Deren Gewichtsbeibehaltung wird dann dadurch bewertet, dass man 10 g der stabilisierten Ausrüstung in eine gewogene Schale einbringt, diese in einen vorgewärmten Umluftofen bei 230°C stellt und die Proben von Zeit zu Zeit (nach Abkühlung in einem Exsiccator) zurückwägt. Der verbliebene Prozentsatz im Vergleich zur Kontrolle gilt als Mass der Hitzebeständigkeit.

| Antioxydans | Gewichtsbeibehaltung (%) | |
|---|---|---|
| | 1 Stunde | 2 Stunden |
| - (Kontrolle) | 41,5 | 34,7 |
| Bsp. 1 | 78,5 | 64,4 |
| Bsp. 2 | 70,2 | 58,4 |
| Bsp. 3 | 68,9 | 56,2 |
| Bsp. 4 | 67,5 | 51,9 |
| Bsp. 6 | 75,7 | 61,1 |

Zusammenfassend ist es ersichtlich, dass vorliegende Erfindung eine neuartige Klasse von Stabilisatorverbindungen zur Verfügung stellt. Die Anteile, Arbeitsweisen und Materialien lassen sich dabei variieren, ohne den Rahmen der wie in den nachfolgenden Ansprüchen definierten Erfindung zu verlassen.

Patentansprüche

1. Verbindungen der Formel

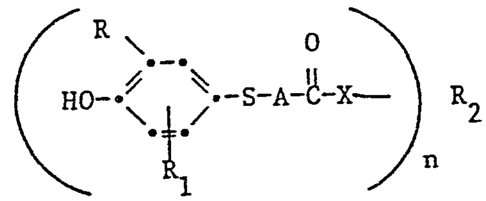

worin R für unsubstituiertes oder durch Aryl, Hydroxyaryl oder Alkaryl substituiertes Alkyl mit 1 bis 18 Kohlenstoffatomen, $R_1$ für Wasserstoff oder R, A für Alkylen mit 2 bis 12 Kohlenstoffatomen, wobei 2 bis 6 Kohlenstoffatome in der geraden Kette zwischen S und $\overset{\overset{O}{\|}}{C}$ sind und X für Sauerstoff oder Schwefel stehen, n 1 bis 6 ist und $R_2$ bei n=1 Alkyl mit 12 bis 30 Kohlenstoffatomen, Phenyl oder niederalkylsubstituiertes Benzyl und bei n=2-6 Alkylen mit 2 bis 18 Kohlenstoffatomen, Arylen, Biphenylen, hydroxysubstituiertes Arylen, Alkanpolyyl mit 3 bis 6 Kohlenstoffatomen oder Polyoxyalkylen mit 2 bis 4 Kohlenstoffatomen in der wiederkehrenden Einheit bedeutet.

2. Verbindungen nach Anspruch 1, dadurch gekennzeichnet, dass sich R und $R_1$ in ortho-Stellung zur Hydroxylgruppe befinden.

3. Verbindungen nach Anspruch 2, dadurch gekennzeichnet, dass R und $R_1$ für Alkyl mit 1 bis 8 Kohlenstoffatomen stehen.

4. Verbindungen nach Anspruch 3, dadurch gekennzeichnet, dass R und $R_1$ für tert.-Butyl stehen.

5. Verbindungen nach Anspruch 1, dadurch gekennzeichnet, dass n 1 ist.

6. Verbindungen nach Anspruch 1, dadurch gekennzeichnet, dass n 2 bis 6 ist.

7. Pentaerythrit-tetrakis-[3-(3,5-di-tert.-butyl-4-hydroxyphenylthio)-propionat] nach Anspruch 6.

8. 2,2-Dimethyl-1,3-propandiol-bis-[3-(3,5-di-tert.-butyl-4-hydroxyphenylthio)-propionat nach Anspruch 6.

9. n-Octadecyl-3-(3,5-di-tert.-butyl-4-hydroxyphenylthio)-propionat nach Anspruch 5.

10. 1,6-Hexandiol-bis-(3,5-di-tert.-butyl-4-hydroxyphenylthio)-propionat nach Anspruch 6.

11. Tetraäthylenglykol-bis-[3-(3,5-di-tert.-butyl-4-hydroxyphenylthio)-propionat] nach Anspruch 6.

12. Trimethylolpropan-tris-[3-(3,5-di-tert.-butyl-4-hydroxyphenylthio)-propionat] nach Anspruch 6.

13. Stoffszusammensetzung, enthaltend ein dem oxydativen, thermischen und aktinischen Abbau unterliegendes organisches Material, und eine wirksame Menge einer Verbindung nach Anspruch 1 oder 6.

14. Zusammensetzung nach Anspruch 13, dadurch gekennzeichnet, dass als organisches Material ein synthetisches Polymer vorliegt.

15. Zusammensetzung nach Anspruch 14, dadurch gekennzeichnet, dass das Polymer unter schlagzähem Polystyrol, Acrylnitril/Butadien/Styrol, Styrol/Butadienkautschuk, Polyestern und Poly-α-olefinen ausgewählt ist.

16. Zusammensetzung nach Anspruch 13, dadurch gekennzeichnet, dass als organisches Material ein Schmieröl vorliegt.

Europäisches Patentamt

**EUROPÄISCHER RECHERCHENBERICHT**

0079855
Nummer der Anmeldung

EP 82 81 0476

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | CHEMICAL ABSTRACTS, Band 87, Nr. 14, 3. Oktober 1977, Seite 16, Nr. 102880a, Columbus, Ohio, USA, A.I. MEDVEDEV et al.: "Standard methods of producing sulfur-containing stabilizers" & IZV. VYSSH. UCHEBN. ZAVED., KHIM. KHIM. TEKHNOL. 1977, 20(4), 568-574 & CHEMICAL SUBSTANCE INDEX, Pq-Z, Juli-Dezember, 1977, Seite 4513CS, Spalte 1, Band 87 * Zusammenfassung * | 1-5,9 | C 07 C 149/40<br>C 09 K 15/14<br>C 08 K 5/36 |
| X | CHEMICAL ABSTRACTS, Band 86, Nr. 18, 2. Mai 1977, Seite 34, Nr. 122248h, Columbus, Ohio, USA & JP - A - 76 142 097 (TEIJIN, LTD.) 07.12.1976 * Zusammenfassung * | 1-4,6, 8,13-15 | RECHERCHIERTE SACHGEBIETE (Int. Cl. 3) |
| X | US-A-3 536 661 (H.J. HAGEMEYER et al.) * Spalte 14, Verbindung XII * | 1 | C 07 C 149/00<br>C 09 K 15/00<br>C 08 K 5/00 |

Der vorliegende Recherchenbericht wurde für alle Patentansprüche erstellt.

| Recherchenort | Abschlußdatum der Recherche | Prüfer |
|---|---|---|
| DEN HAAG | 20-01-1983 | GRAMAGLIA R. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument